# EUROPEAN PATENT SPECIFICATION

(11) **EP 0 573 551 B1**
(45) Date of publication and mention of the grant of the patent: **21.05.2003**
(21) Application number: 92907232.0
(22) Date of filing: 27.02.1992
(51) Int. Cl.: C12N 15/12, C12N 15/62

(54) **SERINE-RICH PEPTIDE LINKERS**
SERIN-REICHE PEPTIDLINKER
SEGMENTS DE LIAISON PEPTIDIQUE RICHES EN SERINE

(30) Priority: 27.02.1991 US 662226
(43) Date of publication of application: 15.12.1993
(73) Proprietor: Micromet AG, 81477 München (DE)
(72) Inventor: HUSTON, James, S., Chestnut Hill, MA 02167 (US); OPPERMANN, Hermann, Medway, MA 02053 (US); TIMASHEFF, Serge, N., Wellesley Hills, MA 02181 (US)
(74) Representative: Dehmel, Albrecht, Dr.
(86) International application number: US9201478
(87) International publication number: WO92015682

(56) References cited:
- WO-A-93/16185
- US-A- 831 967
- BIOCHEMICAL AND BIOPHYSICAL RESEARCH COMMUNICATIONS. vol. 171, no. 1 , 31 August 1990 , DULUTH, MINNESOTA US pages 1 - 6 BATRA JK;CHAUDHARY VK;FITZGERALD D;PASTAN I; 'TGF alpha-anti-Tac(Fv)-PE40: a bifunctional toxin cytotoxic for cells with EGF or IL2 receptors.'
- SCIENCE vol. 242 , October 1988 , LANCASTER, PA pages 423 - 430 BIRD RE;HARDMAN KD;JACOBSON JW;JOHNSON S;KAUFMAN BM;LEE SM;LEE T;POPE SH;RIORDAN GS;WHITLOW M; 'Single-chain antigen-binding proteins'
- JOURNAL OF BIOLOGICAL CHEMISTRY. vol. 265, no. 25 , 5 September 1990 , BALTIMORE US pages 15198 - 15202 BATRA JK;FITZGERALD D;GATELY M;CHAUDHARY VK;PASTAN I; 'Anti-Tac(Fv)-PE40, a single chain antibody Pseudomonas fusion protein directed at interleukin 2 receptor bearing cells.'
- JOURNAL OF BIOLOGICAL CHEMISTRY. vol. 265, no. 30 , 25 October 1990 , BALTIMORE US pages 18615 - 18620 BEDZYK WD;WEIDNER KM;DENZIN LK;JOHNSON LS;HARDMAN KD;PANTOLIANO MW;ASEL ED;VOSS EW JR; 'Immunological and structural characterization of a high affinity anti-fluorescein single-chain antibody.'
- Nature, Vol. 339, issued 01 June 1989, CHAUDHARY et al., "A recombinant immunotoxin consisting of two antibody variable domains fused to Pseudomonas exotoxin", pages 394 to 397, see entire document.

### SEQUENCE LISTING

(1) GENERAL INFORMATION:
   (i) APPLICANT: HUSTON, JAMES S
      OPPERMANN, HERMANN
      TIMASHEFF, SERGE N
   (ii) TITLE OF INVENTION: SERINE RICH PEPTIDE LINKER
   (iii) NUMBER OF SEQUENCES: 9
   (iv) CORRESPONDENCE ADDRESS:
      (A) ADDRESSEE: CREATIVE BIOHOLECULES, INC./PATENT DEPT.
      (B) STREET: 35 SOUTH STREET
      (C) CITY: HOPKINTON
      (D) STATE: MA
      (E) COUNTRY: USA
      (F) ZIP: 01748
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 07/662,226
      (B) FILING DATE: 27-FEB-1991
   (viii) ATTORNEY/AGENT INFORMATION:
      (A) NAME: CAMPBELL ESQ, PAULA A
      (B) REGISTRATION NUMBER: 32,503
      (C) REFERENCE/DOCKET NUMBER: CRP-064PC
   (ix) TELECOMMUNICATION INFORMATION:
      (A) TELEPHONE: 617/248-7000 (ATTY)
(2) INFORMATION FOR SEQ ID NO:1:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Peptide
      (B) LOCATION: 1..13
      (D) OTHER INFORMATION: /note= "(SER)4-GLY LINKER. THE REPEATING SEQUENCE "(SER)4-GLY" (E.G., RES. 3-7) MAY BE REPEATED MULTIPLE TIMES (SEE SPECIFICATION.)
   (xi) SEQUENCE DESCRIPTION: SEQ ID NO:1:
   Ser Gly Ser Ser Ser Ser Gly Ser Ser Ser Ser Gly Ser
   1               5                   10
(2) INFORMATION FOR SEQ ID NO:2:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: misc_feature
      (B) LOCATION: 1..36
      (D) OTHER INFORMATION: /note= "LINKER SEQUENCE (TOP STRAND)"
   (xi) SEQUENCE DESCRIPTION: SEQ ID NO:2:
   CCTCCGGATC TTCATCTAGC GGTTCCAGCT CGAGTG                      36
(2) INFORMATION FOR SEQ ID NO:3:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Peptide
      (B) LOCATION: 1..13
      (D) OTHER INFORMATION: /note= "(XAA)4-GLY LINKER, WHERE RES.3-7 ARE THE REPEATING UNIT AND UP TO 2 OF THE XAA'S IN REPEAT UNIT CAN BE THR, THE REMAINDER SER.
   (xi) SEQUENCE DESCRIPTION: SEQ ID NO:3:
   Xaa Gly Xaa Xaa Xaa Xaa Gly Xaa Xaa Xaa Xaa Gly Xaa
   1               5                   10
(2) INFORMATION FOR SEQ ID NO:4:
   (i) SEQUENCE CHARACTERISTICS:
      (A) LENGTH: 1110 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1101
   (xi) SEQUENCE DESCRIPTION: SEQ ID NO:4:
   ATG AAA GCA ATT TTC GTA CTG AAA GGT TCA CTG GAC AGA GAT CTG GAC       48
   Met Lys Ala Ile Phe Val Leu Lys Gly Ser Leu Asp Arg Asp Leu Asp
    1               5                  10                  15
   TCT CGT CTG GAT CTG GAC GTT CGT ACC GAC CAC AAA GAC CTG TCT GAT       96
   Ser Arg Leu Asp Leu Asp Val Arg Thr Asp His Lys Asp Leu Ser Asp
               20                  25                  30
   CAC CTG GTT CTG GTC GAC CTG GCT CGT AAC GAC CTG GCT CGT ATC GTT      144
   His Leu Val Leu Val Asp Leu Ala Arg Asn Asp Leu Ala Arg Ile Val
           35                  40                  45
   ACT CCC GGG TCT CGT TAC GTT GCG GAT CTG GAA TTC ATG GCT GAC AAC      192
   Thr Pro Gly Ser Arg Tyr Val Ala Asp Leu Glu Phe Met Ala Asp Asn
       50                  55                  60
   AAA TTC AAC AAG GAA CAG CAG AAC GCG TTC TAC GAG ATC TTG CAC CTG      240
   Lys Phe Asn Lys Glu Gln Gln Asn Ala Phe Tyr Glu Ile Leu His Leu
   65                  70                  75                  80
   CCG AAC CTG AAC GAA GAG CAG CGT AAC GGC TTC ATC CAA AGC CTG AAA      288
   Pro Asn Leu Asn Glu Glu Gln Arg Asn Gly Phe Ile Gln Ser Leu Lys
                   85                  90                  95
   GAA GAG CCG TCT CAG TCT GCG AAT CTG CTA GCG GAT GCC AAG AAA CTG      336
   Glu Glu Pro Ser Gln Ser Ala Asn Leu Leu Ala Asp Ala Lys Lys Leu
               100                 105                 110
   AAC GAT GCG CAG GCA CCG AAA TCG GAT CCC GAA GTT CAA CTG CAA CAG      384
   Asn Asp Ala Gln Ala Pro Lys Ser Asp Pro Glu Val Gln Leu Gln Gln
           115                 120                 125
   TCT GGT CCT GAA TTG GTT AAA CCT GGC GCC TCT GTG CGC ATG TCC TGC        432
   Ser Gly Pro Glu Leu Val Lys Pro Gly Ala Ser Val Arg Met Ser Cys
       130                 135                 140
   AAA TCC TCT GGG TAC ATT TTC ACC GAC TTC TAC ATG AAT TGG GTT CGC        480
   Lys Ser Ser Gly Tyr Ile Phe Thr Asp Phe Tyr Met Asn Trp Val Arg
   145                 150                 155                 160
   CAG TCT CAT GGT AAG TCT CTA GAC TAC ATC GGG TAC ATT TCC CCA TAC        528
   Gln Ser His Gly Lys Ser Leu Asp Tyr Ile Gly Tyr Ile Ser Pro Tyr
                   165                 170                 175
   TCT GGG GTT ACC GGC TAC AAC CAG AAG TTT AAA GGT AAG GCG ACC CTT        576
   Ser Gly Val Thr Gly Tyr Asn Gln Lys Phe Lys Gly Lys Ala Thr Leu
               180                 185                 190
   ACT GTC GAC AAA TCT TCC TCA ACT GCT TAC ATG GAG CTG CGT TCT TTG        624
   Thr Val Asp Lys Ser Ser Ser Thr Ala Tyr Met Glu Leu Arg Ser Leu
           195                 200                 205
   ACC TCT GAG GAC TCC GCG GTA TAC TAT TGC GCG GGC TCC TCT GGT AAC        672
   Thr Ser Glu Asp Ser Ala Val Tyr Tyr Cys Ala Gly Ser Ser Gly Asn
       210                 215                 220
   AAA TGG GCC ATG GAT TAT TGG GGT CAT GGT GCT AGC GTT ACT GTG AGC        720
   Lys Trp Ala Met Asp Tyr Trp Gly His Gly Ala Ser Val Thr Val Ser
   225                 230                 235                 240
   TCC TCC GGA TCT TCA TCT AGC GGT TCC AGC TCG AGT GGA TCC GAC GTC        768
   Ser Ser Gly Ser Ser Ser Ser Gly Ser Ser Ser Ser Gly Ser Asp Val
                   245                 250                 255
   GTA ATG ACC CAG ACT CCG CTG TCT CTG CCG GTT TCT CTG GGT GAC CAG        816
   Val Met Thr Gln Thr Pro Leu Ser Leu Pro Val Ser Leu Gly Asp Gln
               260                 265                 270
   GCT TCT ATT TCT TGC CGC TCT TCC CAG TCT CTG GTC CAT TCT AAT GGT        864
   Ala Ser Ile Ser Cys Arg Ser Ser Gln Ser Leu Val His Ser Asn Gly
           275                 280                 285
   AAC ACT TAC CTG AAC TGG TAC CTG CAA AAG GCT GGT CAG TCT CCG AAG        912
   Asn Thr Tyr Leu Asn Trp Tyr Leu Gln Lys Ala Gly Gln Ser Pro Lys
       290                 295                 300
   CTT CTG ATC TAC AAA GTC TCT AAC CGC TTC TCT GGT GTC CCG GAT CGT        960
   Leu Leu Ile Tyr Lys Val Ser Asn Arg Phe Ser Gly Val Pro Asp Arg
   305                 310                 315                 320
   TTC TCT GGT TCT GGT TCT GGT ACT GAC TTC ACC CTG AAG ATC TCT CGT       1008
   Phe Ser Gly Ser Gly Ser Gly Thr Asp Phe Thr Leu Lys Ile Ser Arg
                   325                 330                 335
   GTC GAG GCC GAA GAC CTG GGT ATC TAC TTC TGC TCT CAG ACT ACT CAT 1056
   Val Glu Ala Glu Asp Leu Gly Ile Tyr Phe Cys Ser Gln Thr Thr His
   340 345 350
   GTA CCG CCG ACT TTT GGT GGT GGC ACC AAG CTC GAG ATT AAA CGT 1101
   Val Pro Pro Thr Phe Gly Gly Gly Thr Lys Leu Glu Ile Lys Arg
   355 360 365
   TAACTGCAG 1110
(2) INFORMATION FOR SEQ ID NO:5:
   (i) SEQUENCE CHARACTERISTICS:
      (A) LENGTH: 367 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Lys Ala Ile Phe Val Leu Lys Gly Ser Leu Asp Arg Asp Leu Asp
   1 5 10 15
   Ser Arg Leu Asp Leu Asp Val Arg Thr Asp His Lys Asp Leu Ser Asp
   20 25 30
   His Leu Val Leu Val Asp Leu Ala Arg Asn Asp Leu Ala Arg Ile Val
   35 40 45
   Thr Pro Gly Ser Arg Tyr Val Ala Asp Leu Glu Phe Met Ala Asp Asn
   50 55 60
   Lys Phe Asn Lys Glu Gln Gln Asn Ala Phe Tyr Glu Ile Leu His Leu
   65 70 75 80
   Pro Asn Leu Asn Glu Glu Gln Arg Asn Gly Phe Ile Gln Ser Leu Lys
   85 90 95
   Glu Glu Pro Ser Gln Ser Ala Asn Leu Leu Ala Asp Ala Lys Lys Leu
   100 105 110
   Asn Asp Ala Gln Ala Pro Lys Ser Asp Pro Glu Val Gln Leu Gln Gln
   115 120 125
   Ser Gly Pro Glu Leu Val Lys Pro Gly Ala Ser Val Arg Met Ser Cys
   130 135 140
   Lys Ser Ser Gly Tyr Ile Phe Thr Asp Phe Tyr Met Asn Trp Val Arg
   145 150 155 160
   Gln Ser His Gly Lys Ser Leu Asp Tyr Ile Gly Tyr Ile Ser Pro Tyr
   165 170 175
   Ser Gly Val Thr Gly Tyr Asn Gln Lys Phe Lys Gly Lys Ala Thr Leu
   180 185 190
   Thr Val Asp Lys Ser Ser Ser Thr Ala Tyr Met Glu Leu Arg Ser Leu
   195 200 205
   Thr Ser Glu Asp Ser Ala Val Tyr Tyr Cys Ala Gly Ser Ser Gly Asn
   210 215 220
   Lys Trp Ala Met Asp Tyr Trp Gly His Gly Ala Ser Val Thr Val Ser
   225 230 235 240
   Ser Ser Gly Ser Ser Ser Ser Gly Ser Ser Ser Ser Gly Ser Asp Val
   245 250 255
   Val Met Thr Gln Thr Pro Leu Ser Leu Pro Val Ser Leu Gly Asp Gln
   260 265 270
   Ala Ser Ile Ser Cys Arg Ser Ser Gln Ser Leu Val His Ser Asn Gly
   275 280 285
   Asn Thr Tyr Leu Asn Trp Tyr Leu Gln Lys Ala Gly Gln Ser Pro Lys
   290 295 300
   Leu Leu Ile Tyr Lys Val Ser Asn Arg Phe Ser Gly Val Pro Asp Arg
   305 310 315 320
   Phe Ser Gly Ser Gly Ser Gly Thr Asp Phe Thr Leu Lys Ile Ser Arg
   325 330 335
   Val Glu Ala Glu Asp Leu Gly Ile Tyr Phe Cys Ser Gln Thr Thr His
   340 345 350
   Val Pro Pro Thr Phe Gly Gly Gly Thr Lys Leu Glu Ile Lys Arg
   355 360 365
(2) INFORMATION FOR SEQ ID NO:6:
   (i) SEQUENCE CHARACTERISTICS:
      (A) LENGTH: 747 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..747
   (xi) SEQUENCE DESCRIPTION: SEQ ID NO:6:
   ATG GAA GTT CAA CTG CAA CAG TCT GGT CCT GAA TTG GTT AAA CCT GGC        48
   Met Glu Val Gln Leu Gln Gln Ser Gly Pro Glu Leu Val Lys Pro Gly
    1               5                  10                  15
   GCC TCT GTG CGC ATG TCC TGC AAA TCC TCT GGG TAC ATT TTC ACC GAC        96
   Ala Ser Val Arg Met Ser Cys Lys Ser Ser Gly Tyr Ile Phe Thr Asp
               20                  25                  30
   TTC TAC ATG AAT TGG GTT CGC CAG TCT CAT GGT AAG TCT CTA GAC TAC       144
   Phe Tyr Met Asn Trp Val Arg Gln Ser His Gly Lys Ser Leu Asp Tyr
           35                  40                  45
   ATC GGG TAC ATT TCC CCA TAC TCT GGG GTT ACC GGC TAC AAC CAG AAG       192
   Ile Gly Tyr Ile Ser Pro Tyr Ser Gly Val Thr Gly Tyr Asn Gln Lys
       50                  55                  60
   TTT AAA GGT AAG GCG ACC CTT ACT GTC GAC AAA TCT TCC TCA ACT GCT       240
   Phe Lys Gly Lys Ala Thr Leu Thr Val Asp Lys Ser Ser Ser Thr Ala
   65                  70                  75                  80
   TAC ATG GAG CTG CGT TCT TTG ACC TCT GAG GAC TCC GCG GTA TAC TAT       288
   Tyr Met Glu Leu Arg Ser Leu Thr Ser Glu Asp Ser Ala Val Tyr Tyr
                   85                  90                  95
   TGC GCG GGC TCC TCT GGT AAC AAA TGG GCG ATG GAT TAT TGG GGT CAT       336
   Cys Ala Gly Ser Ser Gly Asn Lys Trp Ala Met Asp Tyr Trp Gly His
               100                 105                 110
   GGT GCT AGC GTT ACT GTG AGC TCC TCC GGA TCT TCA TCT AGC GGT TCC       384
   Gly Ala Ser Val Thr Val Ser Ser Ser Gly Ser Ser Ser Ser Gly Ser
           115                 1120                125
   AGC TCG AGT GGA TCC GAC GTC GTA ATG ACC CAG ACT CCG CTG TCT CTG       432
   Ser Ser Ser Gly Ser Asp Val Val Met Thr Gln Thr Pro Leu Ser Leu
       130                 135                 140
   CCG GTT TCT CTG GGT GAC CAG GCT TCT ATT TCT TGC CGC TCT TCC CAG       480
   Pro Val Ser Leu Gly Asp Gln Ala Ser Ile Ser Cys Arg Ser Ser Gln
   145                 150                 155                 160
   TCT CTG GTC CAT TCT AAT GGT AAC ACT TAC CTG AAC TGG TAC CTG CAA       528
   Ser Leu Val His Ser Asn Gly Asn Thr Tyr Leu Asn Trp Tyr Leu Gln
                   165                 170                 175
   AAG GCT GGT CAG TCT CCG AAG CTT CTG ATC TAC AAA GTC TCT AAC CGC       576
   Lys Ala Gly Gln Ser Pro Lys Leu Leu Ile Tyr Lys Val Ser Asn Arg
               180                 185                 190
   TTC TCT GGT GTC CCG GAT CGT TTC TCT GGT TCT GGT TCT GGT ACT GAC       624
   Phe Ser Gly Val Pro Asp Arg Phe Ser Gly Ser Gly Ser Gly Thr Asp
           195                 200                 205
   TTC ACC CTG AAG ATC TCT CGT GTC GAG GCC GAA GAC CTG GGT ATC TAC        672
   Phe Thr Leu Lys Ile Ser Arg Val Glu Ala Glu Asp Leu Gly Ile Tyr
       210                 215                 220
   TTC TGC TCT CAG ACT ACT CAT GTA CCG CCG ACT TTT GGT GGT GGC ACC        720
   Phe Cys Ser Gln Thr Thr His Val Pro Pro Thr Phe Gly Gly Gly Thr
   225                 230                 235                 240
   AAG CTC GAG ATT AAA CGT TAA CTG CAG                                    747
   Lys Leu Glu Ile Lys Arg
                   245
(2) INFORMATION FOR SEQ ID NO:7:
   (i) SEQUENCE CHARACTERISTICS:
      (A) LENGTH: 249 amino acids
      (B) TYPE: amino acid
      (D) TOPOLOGY: linear
   (ii) MOLECULE TYPE: protein
   (xi) SEQUENCE DESCRIPTION: SEQ ID NO:7:
(2) INFORMATION FOR SEQ ID NO:8:
   (i) SEQUENCE CHARACTERISTICS:
      (A) LENGTH: 1416 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1416
   (xi) SEQUENCE DESCRIPTION: SEQ ID NO:8:
(2) INFORMATION FOR SEQ ID NO:9:
   (i) SEQUENCE CHARACTERISTICS:
      (A) LENGTH: 471 amino acids
      (B) TYPE: amino acid
      (D) TOPOLOGY: linear
   (ii) MOLECULE TYPE: protein
   (xi) SEQUENCE DESCRIPTION: SEQ ID NO:9

## Description

The present invention is in the fields of peptide linkers, fusion proteins and single-chain antibodies.

Two or more polypeptides may be connected to form a fusion protein. This is accomplished most readily by fusing the parent genes that encode the proteins of interest. Production of fusion proteins that recover the functional activities of the parent proteins may be facilitated by connecting genes with a bridging DNA segment encoding a peptide linker that is spliced between the polypeptides connected in tandem. The present invention addresses a novel class of linkers that confer unexpected and desirable qualities on the fusion protein products.

An example of one variety of such fusion proteins is an antibody binding site protein also known as a single-chain Fv (sFv) which incorporates the complete antibody binding site in a single polypeptide chain. Antibody binding site proteins can be produced by connecting the heavy chain variable region (V_{H}) of an antibody to the light chain variable region (V_{L}) by means of a peptide linker. See, PCT International Publication No. WO 88/09344. Such sFv proteins have been produced to date that faithfully reproduce the binding affinities and specificities of the parent monoclonal antibody. However, there have been some drawbacks associated with them, namely, that some sFv fusion proteins have tended to exhibit low solubility in physiologically acceptable media. For example, the anti-digoxin 26-10 sFv protein, which binds to the cardiac glycoside digoxin, can be refolded in 0.01M NaOAc buffer, pH 5.5, to which urea is added to a final concentration of 0.25M to produce approximately 22% active anti-digoxin sFv protein. The anti-digoxin sFv is inactive as a pure protein in phosphate buffered saline (PBS) which is a standard buffer that approximates the ionic strength and neutral pH conditions of human serum. In order to retain digoxin binding activity in PBS the 26-10 sFv must be stored in 0.01 M sodium acetate, pH 5.5, 0.25 M urea diluted to nanomolar concentrations in PBS containing 1% horse serum or 0.1% gelatin, a concentration which is too low for most therapeutic or pharmaceutical use.

Therefore, it is an object of the invention to design and prepare fusion proteins which are 1) soluble at high concentrations in physiological media, and 2) resistant to proteolytic degredation.

The present invention relates to a peptide linker comprising a large proportion of serine residues which, when used to connect two polypeptide domains, produces a fusion protein which has increased solubility in aqueous media and improved resistance to proteolysis. In one aspect, the invention provides a family of biosynthetic proteins comprising first and second protein domains which are biologically active individually or act together to effect biological activity, wherein the domains are connected by a peptide linker comprising the sequence (X, X, X, X, Gly)_{y} wherein y typically is 2 or greater, up to two Xs in each unit are Thr, and the remaining Xs in each unit are Ser. Preferably, the linker takes the form (Ser, Ser, Ser, Ser, Gly)_{y} where Y is greater than 1. The linker preferably comprises at least 75 percent serine residues.

The linker can be used to prepare single chain binding site proteins wherein one of the protein domains attached to the linker comprises or mimicks the structure of an antibody heavy chain variable region and the other domain comprises or mimicks the structure of an antibody light chain variable domain. A radioactive isotope advantageously may be attached to such structures to produce a family of imaging agents having high specificity for target structure dictated by the particular affinity and specificity of the single chain binding site. Alternatively, the linker may be used to connect a polypeptide ligand and a polypeptide effector. For example, a ligand can be a protein capable of binding to a receptor or adhesion molecule on a cell in vivo, and the effector a protein capable of affecting the metabolism of the cell. Examples of such constructs include those wherein the ligand is itself a single chain immunoglobulin binding site or some other form of binding protein or antibody fragment, and the effector is, for example, a toxin.

Preferred linkers for sFv comprise between 8 and 40 amino acids, more preferably 10-15, most preferably 13, wherein at least 40%, and preferably 50% are serine. Glycine is a preferred amino acid for remaining residues; threonine may also be used; and preferably, charged residues are avoided.

Fusion proteins containing the serine-rich peptide linker are also the subject of the present invention, as are DNAs encoding the proteins, cells expressing them, and method of making them.

The serine-rich peptide linkers of the present invention can be used to connect the subunit polypeptides of a biologically active protein, that is, linking one polypeptide domain with another polypeptide domain, thereby forming a biologically active fusion protein; or to fuse one biologically active polypeptide to another biologically active peptide, thereby forming a bifunctional fusion protein expressing both biological activities. A particularly effective linker for forming this protein contains the following amino acid sequence (sequence ID No. 1):

The serine-rich linkers of the present invention produce proteins which are biologically active and which remain in solution at a physiologically acceptable pH and ionic strength at much higher concentrations than would have been predicted from experience. The serine-rich peptide linkers of the present invention often can provide significant improvements in refolding properties of the fusion protein expressed in procaryotes. The present serine-rich linkers are resistant to proteolysis, thus fusion proteins which are relatively stable in vivo can be made using the present linker and method. In particular, use of the linkers of the present invention to fuse domains mimicking V_{H} and V_{L} from monoclonal antibody results in single chain binding site proteins which dissolve in physiological media, retain their activity at high concentrations, and resist lysis by endogenous proteases.

### Detailed Description of the Invention

The serine-rich peptide linkers of the present invention are used to link through peptide bonded structure two or more polypeptide domains. The polypeptide domains individually may be biologically active proteins or active polypeptide segments, for example, in which case a multifunctional protein is produced. Alternatively, the two domains may interact cooperatively to effect the biological function. The resulting protein containing the linker(s) is referred to herein as a fusion protein.

The preferred length of a serine-rich peptide of the present invention depends upon the nature of the protein domains to be connected. The linker must be of sufficient length to allow proper folding of the resulting fusion protein. The length required can be estimated as follows:
1. Single-Chain Fv (sFv). For a single chain antibody binding site comprising mimicks of the light and heavy chain variable regions of an antibody protein (hereinafter, sFv), the linker preferably should be able to span the 3.5 nanometer (nm) distance between its points of covalent attachment between the C-terminus of one and the N-terminus of the other V domain without distortion of the native Fv conformation. Given the 0.38 nm distance between adjacent peptide bonds, a preferred linker should be at least about 10 residues in length. Most preferable, a 13-15 amino acid residue linker is used in order to avoid conformational strain from an overly short connection, while avoiding steric interference with the combining site from an excessively long peptide.
2. Connecting domains in a dimeric or multimeric protein for which a 3-dimensional conformation is known. Given a 3-dimensional structure of the protein of interest, the minimum surface distance between the chain termini to be bridged, d (in nanometers), should be determined, and then the approximate number of residues in the linker, n, is calculated by dividing d by 0.38 nm (the peptide unit length). A preferred length should be defined ultimately by empirically testing linkers of different sizes, but the calculated value provides a good first approximation.
3. Connecting domains in a dimeric or multimeric protein for which no 3-dimensional conformation is known. In the absence of information regarding the protein's 3-dimensional structure, the appropriate linker length can be determined operationally by testing a series of linkers (e.g., 5, 10, 15, 20, or 40 amino acid residues) in order to find the range of usable linker sizes. Fine adjustment to the linker length then can be made by comparing a series of single-chain proteins (e.g., if the usable n values were initially 15 and 20, one might test 14, 15, 16, 17, 18, 19, 20, and 21) to see which fusion protein has the highest specific activity.
4. Connection of independent domains (i.e., independently functional proteinsor polypeptides) or elements of secondary structure (alpha or beta strands). For optimal utility, this application requires empirically testing serine-rich linkers of differing lengths to determine what works well. In general, a preferred linker length will be the smallest compatible with full recovery of the native functions and structures of interest. Linkers wherein 1 ≤ y ≤ 4 work well in many instances.

After the ideal length of the peptide linker is determined, the percentage of serine residues present in the linker can be optimized. As was stated above, preferably at least 75% of a peptide linker of the present invention is serine residues. The currently preferred linker is (SerSerSerSerGly)_{y} [residues 3-7 of sequence ID No. 1] where y comprises an integer from 1 to 5. Additional residues may extend C-terminal or N-terminal of the linker; preferably such additional residues comprising Ser, Thr, or Gly. Up to two of each of the serine residues on each segment may be replaced by Thr, but this has the tendency to decrease the water solubility of the fusion constructs. For constructs wherein the two linked domains cooperate to effect a single biological function, such as an sFv, it is preferred to avoid use of charged residues. Generally, in linkers of more than 10 residues long, any naturally occurring amino acid may be used once, possibly twice, without unduly degrading the properties of the linker.

The serine-rich peptide linker can be used to connect a protein or polypeptide domain with a biologically active peptide, or one biologically active peptide to another to produce a fusion protein having increased solubility, improved folding properties and greater resistance to proteolysis in comparison to fusion proteins using non-serine rich linkers. The linker can be used to make a functional fusion protein from two unrelated proteins that retain the activities of both proteins. For example, a polypeptide toxin can be fused by means of a linker to an antibody, antibody fragment, sFv or peptide ligand capable of binding to a specific receptor to form a fusion protein which binds to the receptor on the cell and kills the cell.

Fusion protein according to the present invention can be produced by amino acid synthesis, if the amino acid sequence is known, or preferably by art-recognized cloning techniques. For example, an oligonucleotide encoding the serine-rich linker is ligated between the genes encoding the domains of interest to form one fused gene encoding the entire single-chain protein. The 5' end of the linker oligonucleotide is fused to the 3' end of the first gene, and the 3' end of the linker is fused to the 5' end of the second gene. Any number of genes can be connected in tandem array to encode multi-functional fusion proteins using the serine-rich polypeptide linker of the present invention. The entire fused gene can be transfected into a host cell by means of an appropriate expression vector.

In a preferred embodiment of the present invention, amino acid sequences mimicking the light (V_{L}) and heavy (V_{H}) chain variable regions of an antibody are linked to form a single chain antibody binding site (sFv) which preferably is free of immunoglobulin constant region. Single chain antibody binding sites are described in detail, for example, in U.S. Patent No. 5,019,513.

A particularly effective serine-rich linker for an sFv protein is a linker having the following amino acid sequence:

That is, in this embodiment y=2; Ser, Gly precedes the modular sequences, and Ser follows them. The serine-rich linker joins the V_{H} with the V_{L} (or vice versa) to produce a novel sFv fusion protein having substantially increased solubility, and resistance to lysis by endogenous proteases.

A preferred genus of linkers comprises a sequence having the formula:

Where up to two Xs in each unit can be Thr, the remaining Xs are Ser, and y in between 1 and 5.

A method for producing a sFv is described in PCT Application No. US88/01737. In general, the gene encoding the variable region from the heavy chain (V_{H}) of an antibody is connected at the DNA level to the variable region of the light chain (V_{L}) by an appropriate oligonucleotide. Upon translation, the resultant hybrid gene forms a single polypeptide chain comprising the two variable domains bridged by a linker peptide.

The sFv fusion protein comprises a single polypeptide chain with the sequence V_{H}- <linker> -V_{L} or V_{L}- <linker> - V_{H}, as opposed to the classical Fv heterodimer of V_{H} and V_{L}. About 3/4 of each variable region polypeptide sequence is partitioned into four framework regions (FRs) that form a scaffold or support structure for the antigen binding site, which is constituted by the remaining residues defining three complementary determining regions (CDRs) which form loops connecting the FRs. The sFv is thus preferably composed of 8 FRs, 6 CDRs, and a linker segment, where the V_{H} sequence can be abbreviated as: and the V_{L} sequence as

The predominant secondary structure in immunoglobulin V regions is the twisted β-sheet. A current interpretation of Fv architecture views the FRs as forming two concentric β-barrels, with the CDR loops connecting antiparallel β-strands of the inner barrel. The CDRs of a given murine monoclonal antibody may be grafted onto the FRs of human Fv regions in a process termed "humanization" or CDR replacement. Humanized antibodies promise minimal immunogenicity when sFv fusion proteins are administered to patients. Humanized single chain biosynthetic antibody binding sites, and how to make and use them, are described in detail in U.S. 5,019,513, as are methods of producing various other FR/CDR chimerics.

The general features of a viable peptide linker for an sFv fusion protein are governed by the architecture and chemistry of Fv regions. It is known that the sFv may be assembled in either domain order, V_{H}-linker-V_{L} or V_{L}-linker-V_{H}, where the linker bridges the gap between the carboxyl (C) and amino (N) termini of the respective domains. For purposes of sFv design, the C-terminus of the amino-terminal V_{H} or V_{L} domain is considered to be the last residue of that sequence which is compactly folded, corresponding approximately to the end of the canonical V region sequence. The amino-terminal V domain is thus defined to be free of switch region residues that link the variable and constant domains of a given H or L chain, which makes the linker sequence an architectural element in sFv structure that corresponds to bridging residues, regardless of their origin. In several examples, fused sFv constructs have incorporated residues from the switch region, even extending into the first constant domain.

In principle, sFv proteins may be constructed to incorporate the Fv region of any monoclonal antibody regardless of its class or antigen specificity. Departures from parent V region sequences may involve changes in CDRs to modify antigen affinity or specificity, or to redefine complementarity, as well as wholesale alteration of framework regions to effect humanization of the sFv or for other purposes. In any event, an effective assay, e.g., a binding assay, must be available for the parent antibody and its sFv analogue. Design of such an assay is well within the skill of the art. Fusion proteins such as sFv immunotoxins intrinsically provide an assay by their toxicity to target cells in culture.

The construction of a single-chain Fv typically is accomplished in two or three phases: (1) isolation of cDNA for the variable regions; (2) modification of the isolated V_{H} and V_{L} domains to permit their joining to form a single chain via a linker; (3) expression of the single-chain Fv protein. The assembled sFv gene may then be progressively altered to modify sFv properties. Escherichia coli (E. coli) has generally been the source of most sFv proteins although other expression systems can be used to generate sFv proteins.

The V_{H} and V_{L} genes for a given monoclonal antibody are most conveniently derived from the cDNA of its parent hybridoma cell line. Cloning of V_{H} and V_{L} from hybridoma cDNA has been facilitated by library construction kits using lambda vectors such as Lambda ZAP^{R} (Stratagene). If the nucleotide and/or amino acid sequences of the V domains are known, then the gene or the protein can be made synthetically. Alternatively, a semisynthetic approach can be taken by appropriately modifying other available cDNA clones or sFv genes by site-directed mutagenesis.

Many alternative DNA probes have been used for V gene cloning from hybridoma cDNA libraries. Probes for constant regions have general utility provided that they match the class of the relevant heavy or light chain constant domain. Unrearranged genomic clones containing the J-segments have even broader utility, but the extent of sequence homology and hybridization stringency may be unknown. Mixed pools of synthetic oligonucleotides based on the J-regions of known amino acid sequence have been used. If the parental myeloma fusion partner was transcribing an endogenous immunoglobulin gene, the authentic clones for the V genes of interest should be distinguished from the genes of endogenous origin by examining their DNA sequences in a Genbank homology search.

The cloning steps described above may be simplified by the use of polymerase chain reaction (PCR) technology. For example, immunoglobulin cDNA can be transcribed from the monoclonal cell line by reverse transcriptase prior to amplification by Tag polymerase using specially designed primers. Primers used for isolation of V genes may also contain appropriate restriction sequences to speed sFv and fusion protein assembly. Extensions of the appropriate primers preferably also should encode parts of the desired linker sequence such that the PCR amplification products of V_{H} and V_{L} genes can be mixed to form the single-chain Fv gene directly. The application of PCR directly to human peripheral blood lymphocytes offers the opportunity to clone human V regions directly in bacteria. See, Davis et al. Biotechnology, 9, (2):165-169 (1991).

Refinement of antibody binding sites is possible by using filamentous bacteriophage that allow the expression of peptides or polypeptides on their surface. These methods have permitted the construction of phage antibodies that express functional sFv on their surface as well as epitope libraries that can be searched for peptides that bind to particular combining sites. With appropriate affinity isolation steps, this sFv-phage methodology offers the opportunity to generate mutants of a given sFv with desired changes in specificity and affinity as well as to provide for a refinement process in successive cycles of modification. See McCafferty et al., Nature, 348:552 (1990), Parmely et al. Gene, 38:305 (1988), Scott et al. Science, 249:386 (1990), Devlin et al. Science, 249:404 (1990), and Cwirla et al., Proc. Nat. Acad. Sci. U.S.A., 87:6378 (1990).

The placement of restriction sites in an sFv gene can be standardized to facilitate the exchange of individual V_{H}, V_{L} linker elements, or leaders (See U.S. 5,019,513, supra). The selection of particular restriction sites can be governed by the choice of stereotypical sequences that may be fused to different sFv genes. In mammalian and bacterial secretion, secretion signal peptides are cleaved from the N-termini of secreted proteins by signal peptidases. The production of sFv proteins by intracellular accumulation in inclusion bodies also may be exploited. In such cases a restriction site for gene fusion and corresponding peptide cleavage site are placed at the N-terminus of either V_{H} or V_{L}. Frequently a cleavage site susceptible to mild acid for release of the fusion leader is chosen.

In a general scheme, a SacI site serves as an adapter at the C-Terminal end of V_{H}. A large number of V_{H} regions end in the sequence -Val-Ser-Ser-, which is compatible with the codons for a SacI site (G AGC TCT), to which the linker may be attached. The linker of the present invention can be arranged such that a -Gly-Ser-is positioned at the C-terminal end of the linker encoded by GGA-TCC to generate a BamHI site, which is useful provided that the same site is not chosen for the beginning of V_{H}.

Alternatively, an XhoI site (CTCGAG) can be placed at the C-terminal end of the linker by including another serine to make a -Gly-Ser-Ser- sequence that can be encoded by GGC-TCG-AGN-, which contains the XhoI site. For sFv genes encoding V_{H}-Linker-V_{L}, typically a PstI site is positioned at the 3' end of the V_{L} following the new stop condon, which forms a standard site for ligation to expression vectors. If any of these restriction sites occur elsewhere in the cDNA, they can be removed by silent base changes using site directed mutagenesis. Similar designs can be used to develop a standard architecture for V_{L}-V_{H} constructions.

Expression of fusion proteins in E. coli as insoluble inclusion bodies provides a reliable method for producing sFv proteins. This method allows for rapid evaluation of the level of expression and activity of the sFv fusion protein while eliminating variables associated with direct expression or secretion. Some fusion partners tend not to interfere with antigen binding which may simplify screening for sFv fusion protein during purification. Fusion protein derived from inclusion bodies must be purified and refolded in vitro to recover antigen binding activity. Mild acid hydrolysis can be used to cleave a labile Asp-Pro peptide bond between the leader and sFv yielding proline at the sFv amino terminus. In other situations, leader cleavage can rely on chemical or enzymatic hydrolysis at specifically engineered sites, such as CNBr cleavage of a unique methionine, hydroxylamine cleavage of the peptide bond between Asn-Gly, and enzymatic digestion at specific cleavage sites such as those recognised by factor Xa, enterokinase or V8 protease.

Direct expression of intracellular sFv proteins which yields the desired sFv without a leader attached is possible for single-chain Fv analogues and sFv fusion proteins. Again, the isolation of inclusion bodies must be followed by refolding and purification. This approach avoids the steps needed for leader removal but direct expression can be complicated by intracellular proteolysis of the cloned protein.

The denaturation transitions of Fab fragments from polyclonal antibodies are known to cover a broad range of denaturant. The denaturation of monoclonal antibody Fab fragments or component domains exhibit relatively sharp denaturation transitions over a limited range of denaturant. Thus, sFv proteins can be expected to differ similarly covering a broad range of stabilities and denaturation properties which appear to be paralleled by their preferences for distinct refolding procedures. Useful refolding protocols include dilution refolding, redox refolding and disulfide restricted refolding. In general, all these procedures benefit from the enhanced solubility conferred by the serine-rich linker of the present invention.

Dilution refolding relies on the observation that fully reduced and denatured antibody fragments can refold upon removal of denaturant and reducing agent with recovery of specific binding activity. Redox refolding utilizes a glutathione redox couple to catalyze disulfide interchange as the protein refolds into its native state. For an sFv protein having a prior art linker such as (GlyGlyGLyGlySer)₃, the protein is diluted from a fully reduced state in 6 M urea into 3 M urea + 25 mM Tris-HCL + 10 mM EDTA, pH 8, to yield a final concentration of approximately 0.1 mg/ml. In a representative protein, the sFv unfolding transition begins around 3 M urea and consequently the refolding buffer represents near-native solvent conditions. Under these conditions, the protein can presumably reform approximations to the V domain structures wherein rapid disulfide interchange can occur until a stable equilibrium is attained. After incubation at room temperature for 16 hours, the material is dialyzed first against urea buffer lacking glutathione and then against 0.01 M sodium acetate + 0.25 M urea, pH 5.5.

In contrast to the sFv protein having the prior art linker described above, with the same sFv protein, but having a serine-rich linker of the present invention, the 3M urea-glutathione refolding solution can be dialyzed directly into 0.05 M potassium phosphate, pH 7, 0.15 NaCl (PBS).

Disulfide restricted refolding offers still another route to obtaining active sFv which involves initial formation of intrachain disulfides in the fully denatured sFv. This capitalizes on the favored reversibility of antibody refolding when disulfides are kept intact. Disulfide crosslinks should restrict the initial refolding pathways available to the molecule as well as other residues adjacent to cysteinyl residues that are close in the native state. For chains with the correct disulfide paring the recovery of a native structure should be favored while those chains with incorrect disulfide pairs must necessarily produce non-native species upon emoval of denaturant. Although this refolding method may give a lower yield than other procedures, it may be able to tolerate higher protein concentrations during refolding.

Proteins secreted into the periplasmic space or into the culture medium appear to refold properly with formation of the correct disulfide bonds. In the majority of cases the signal peptide sequence is removed by a bacterial signal peptidase to generate a product with its natural amino terminus. Even though most secretion systems currently give considerably lower yields than intracellular expression, the rapidity of obtaining correctly folded and active sFv proteins can be of decisive value for protein engineering. The ompA or pelB signal sequence can be used to direct secretion of the sFv.

If some sFv analogues or fusion proteins exhibit lower binding affinities than the parent antibody, further purification of the sFv protein or additional refinement of antigen binding assays may be needed. On the other hand, such sFv behavior may require modification of protein design. Changes at the amino-termini of V domains may on occasion perturb a particular combining site. Thus, if an sFv were to exhibit a lower affinity for antigen than the parent Fab fragment, one could test for a possible N-terminal perturbation effect. For instance, given a V_{L}-V_{H} that was suspect, the V_{H}-V_{L} construction could be made and tested. If the initially observed perturbation were changed or eliminated in the alternate sFv species, then the effect could be traced to the initial sFv design.

The invention will be understood further from the following nonlimiting examples.

### EXAMPLES

### Example 1. Preparation and Evaluation of an Anti-digoxin 26-10 sFv Having a Serine-rich Linker

An anti-digoxin 26-10 sFv containing a serine-rich peptide linker (Sequence No. 1, identified below) of the present invention was prepared as follows:

A set of synthetic oligonucleotides was prepared using phosphoramidite chemistry on a Cruachem DNA synthesizer, model PS250. The nucleotide sequence in the appropriate reading frame encodes the polypeptide from 1-12 while residue 13 is incorporated as part of the Bam Hl site that forms upon fusion to the downstream Ban Hl fragment that encodes V_{L}; and the first serine residue in the linker was attached to a serine at the end of the 26-10 V_{H} region of the antibody. This is shown more clearly in Sequence ID Nos. 4 and 5.

The synthetic oligonucleotide sequence which was used in the cassette mutagenesis was as follows:

The complementary oligomers, when annealed to each other, present a cohesive end of a SacI site upstream and a BamHI site downstream.

The nucleotide sequence was designed to contain useful 6-base restriction sites which will allow combination with other single chain molecules and additional modifications of the leader. The above-described synthetic oligonucleotides were assembled with the V_{H} and V_{L} regions of the anti-digoxin 26-10 gene as follows:
A pUC plasmid containing the 26-10 sFv gene (disclosed in PCT International Publication No. WO 88/09344) containing a (Gly-Gly-Gly-Gly-Ser)ₙ linker between a SacI site at the end of the V_{H} region and a unique BamHI site which had been inserted at the beginning of V_{L} region was opened at SacI and BamHI to release the sequence encoding for the prior art linker and to accept the oligonucleotides defined by Sequence No. 2. The resulting plasmid was called pH899.

The new 26-10 sFv gene of pH899 was inserted into an expression vector, pH895, for fusion with a modified fragment B (MFB) of staphlococcal protein A. (See Sequence ID No. 4.) The modified FB leader has glutamyl resides at positions FB-36 and FB-37 instead of 2 aspartyl residues, which reduces unwanted ancillary cleavage during acid treatment. The modified pH895 is essentially equivalent to pC105 (except for the slightly modified leader) as previously described in Biochemistry, 29(35):8024-8030 (1990). The assembly was done by replacing the old sFv fragment with the new sFv between XbaI (in V_{H}) and PstI (at the end of sFv) in the expression plasmid pH895, opened at unique XbaI and PstI sites. The resulting new expression vector was named pH908. An expression vector utilizing an MLE-MFB leader was constructed as follows.

The mFB-sFv gene was retrieved by treating pH908 with EcoRI and PstI and inserted into a trp expression vector containing the modified trp LE leader peptide (MLE) producing plasmid pH912. This vector resembled essentially the pD312 plasmid as described in PNAS, 85: 5879-5883 (1988) but having removed from it the EcoRI site situated between the Tet-R gene and the SspI site. Plasmid pH912 contained the MLE-mFB-sFv gene shown in sequence 4. The MLE starts at the N-terminus of the protein and ends at the glutamic acid residue, amino acid residue 59. The mFB leader sequence starts at the methionine residue, amino acid residue 61, and ends at the aspartic acid residue, amino acid residue 121. Phenylanine residue 60 is technically part of the Eco RI restriction site sequence at the junction of the MLE and mFB.

Expression of sFv transfected into E. coli (strain JM101) by the plasmid pH912 was under control of the trp promoter. E. coli was transformed by pH912 under selection by tetracycline. Expression was induced in M9 minimal medium by addition of indole acrylic acid (10 µg/ml) at a cell density with A₆₀₀ = 1 resulting in high level expression and formation of inclusion bodies which were harvested from cell paste.

After expression in E. coli of the sFv protein containing the novel linker of the present invention, the resultant cells were suspended in 25 mM Tris-HCl, pH 8, and 10m mM EDTA treated with 0.1% lysozyme overnight, sonicated at a high setting for three 5 minute periods in the cold, and spun in a preparative centrifuge at 11,200 x g for 30 minutes. For large scale preparation of inclusion bodies, the cells are concentrated by ultrafiltration and then lysed with a laboratory homogenizer such as with model 15MR, APV homogenizer manufactured by Gaulin, Inc. The inclusion bodies are then collected by centrifugation. The resultant pellet was then washed with a buffer containing 3 M urea, 25 mM Tris-HC pH8, and 10 mEDTA.

The purification of the 26-10 sFv containing the linker of the present invention from the MLE-mFB-sFv fusion protein was then accomplished according to the following procedure:

### 1) Solubilization of Fusion Protein in Guanidine Hydrochloride

The MLE-mFB-sFv inclusion bodies were weighed and were then dissolved in a 6.7 M GuHCl (guanidine hydrochloride) which had been dissolved in 10% acetic acid. An amount of GuHCl equal to the weight of the recovered inclusion bodies was then added to the solution and dissolved to compensate for the water present in the inclusion body pellet.

### 2) Acid Cleavage of the Unique Asp-Pro Bond at the Junction of the Leader and 26-10 sFv

The Asp-Pro bond (amino acid residues 121 and 122 of Sequence Nos. 4 and 5) was cleaved in the following manner. Glacial acetic acid was added to the solution of step 1 to 10% of the total volume of the solution. The pH of the solution was then adjusted to 2.5 with concentrated HCl. This solution was then incubated at 37°C for 96 hours. The reaction was stopped by adding 9 volumes of cold ethanol, stored at -20°C for several hours, followed by centrifugation to yield a pellet of precipitated 26-10 sFv and uncleaved fusion protein. The heavy chain variable region of the sFv molecule extended from amino acid residue 123 to 241; the linker included amino acid residues 242 to 254; and the variable light region extended from amino acid residue 255 to 367 of Sequence Nos. 4 and 5. Note also that Sequence No. 6 and 7 shows a similar sFv starting with methionine at residues 1 followed by V_{H} (residues 2-120), linker (121-133), and V_{L} (134-246). This gene product was expressed directly by the T7 expression system with formation of inclusion bodies.

### 3) Re-dissolution of Cleavage Products

The precipitated sFv cleavage mixture from step 2 was weighed and dissolved in a solution of 6 M GuHCl + 25 mM Tris HC1 + 10 mM EDTA having a pH of 8.6. Solid GuHCl in an amount equal to the weight of the sFv cleavage mixture from step two was then added and dissolved in the solution. The pH of the solution was then adjusted to 8.6 and dithiothreitol was added to the solution such that the resultant solution contained 10 mM dithiothreitol. The solution was then incubated at room temperature for 5 hours.

### 4) Renaturation of 26-10 sFv

The solution obtained from step 3 was then diluted 70-fold to a concentration of about 0.2mg of protein/ml with a buffer solution containing 3 M urea, 25 mM Tris-HCl, pH 8, 10 mM EDTA 1 mM oxidized gluthathione, 0.1 mM reduced gluthathione, and incubated at room temperature for 16 hours. The resultant protein solution was then dialyzed in the cold against PBSA to complete the refolding of the sFv protein.

### 5) Affinity Purification of the Active Anti-digoxin 26-10 sFv

The refolded protein from step 4 was loaded onto a column containing ouabain-amine-Sepharose 4B, and the column was washed successively with PBSA, followed by two column volumes of 1 M NaCl in PBSA and then again with PBSA to remove salt. Finally, the active protein was displaced from the resin by 20 mM ouabain in PBSA. Absorbance measurements at 280 nm indicated which fractions contained active protein. However, the spectra of the protein and ouabain overlap. Consequently, ouabain was removed by exhaustive dialysis against PBSA in order to accurately quantitate the protein yield.

### 6) Removal of Uncleaved Fusion Protein and the MLE-mFB Leader

Finally, the solution from step 5 containing the active refolded protein (sFv and MLE-mFB-sFv) was chromatographed on an IgG-Sepharose column in PBSA buffer. The uncleaved MLE-mFB-sFv protein bound to the immobilized immunoglobulin and the column effluent contained essentially pure sFv.

In conclusion, the incorporation of a serine-rich peptide linker of 13 residues [Ser-Gly-(Ser-Ser-Ser-Ser-Gly)2-Ser-] in the 26-10 sFv yielded significant improvements over the 26-10 sFv with a glycine-rich linker of 15 residues, [-(Gly-Gly-Gly-Gly-Ser)₃].

The serine-rich peptide linker of the present invention results in a number of improvements over the previous peptide linkers including:
1. Refolding and storage conditions are consistent with normal serum conditions, thereby making applications to pharmacology and toxicology accessible. The 26-10 sFv can be renatured in PBS (0.05 M potassium phosphate, 0.15 M NaCl, pH 7.0); 0.03% azide is added as a bacteriostatic agent for laboratory purposes but would be excluded in any animal or clinical applications. The old linker, 26-10 sFv had to be renatured into 0.01 M sodium acetate, pH 5.5, with 0.25 M urea added to enhance the level of active protein.
2. Solubility was vastly improved from a limit of about 5OD₂₈₀ units per ml (about 3 mg/ml) to 52 OD₂₈₀ units per ml (about 33 mg/ml), and possibly greater in buffers other than PBSA. The highly concentrated protein solution was measured directly with a 0.2 mm path length cell. The protein concentration was estimated by multiplying by 50 the absorptions at 280 nm, subtracting twice the scattering absorbance at 333 nm, which yields a corrected A280 of about 52 units per ml.
3. Fidelity of the antigen binding site was retained by the new serine-rich linker 26-10 sFv, which is consistent with an uncharged linker peptide that has minimal interactions with the V domains.
4. Enhanced stability at normal serum pH and ionic strength. In PSBSA, 26-10 sFv with the (GGGGS)₃ linker loses binding activity irreversibly whereas the 26-10 sFv containing the new serine-rich linker is completely stable in PBSA.
5. Enhanced resistance to proteolysis. The presence of the serine-rich linker improves resistance to endogenous proteases in vivo, which results in a longer plasma/half-life of the fusion protein.

### Example 2. Preparation of a Fusion Protein Having a Serine Rich Linker

A fusion protein was prepared containing a serine rich linker linking two unrelated proteins. A fusion gene was constructed as described in Example 1 above, except that in lieu of the V_{L} and V_{H} genes described in Example 1, genes encoding the following proteins were fused: the dominant dhfr gene (Sequence No. 8, residues 1-576) and the neo gene (Sequence No. 8, residues 621-1416) were fused with a linker having the sequence:

The four residues SVTV (numbers 189-192 of Seq. ID No. 8) can be regarded as part of the linker. These were left over from the sFv from which the linker sequences used in this example was derived. The resulting protein was a functional fusion protein encoding domains from two unrelated proteins which retained the activity of both. Thus, this DNA included on a plasmid inparts to successfully transfected cells resistance to both methotrexate, due to the action of the DHFR enzyme, and to neomycin, due to the action of the neo expression product.

## Claims

1. A biosynthetic protein comprising first and second protein domains biologically active individually or together, said domains being connected by a peptide linker consisting of between 8 and 40 amino acid residues with at least 50% of the residues being serine, excludinq Ser Ile (Ser Ser Ser Ser Gly)₃ Ser Asp Ile.

2. The biosynthetic protein according to claim 1, wherein the peptide linker comprises the sequence (Ser Ser Ser Ser Gly)_{y} where y is greater than 1.

3. The protein according to claim 1 or 2 wherein:
(i) one of said protein domains comprises an antibody heavy chain variable region (VH) and the other said protein domains comprises an antibody light chain variable regions (VL), said protein optionally being labelled with a radioactive isotope; or
(ii) the first protein domain comprises a polypeptide ligand and the second domain comprises a polypeptide effector, said ligand being capable of binding to a receptor or adhesion molecule on a cell and said effector being capable of affecting the metabolism of the cell, and for example the ligand is an sFv fusion protein, or an antibody fragment, and for example the effector is a toxin; or
(iii) one of said protein domains has the structure of a V_{L} and the other of said protein domains has the structure of a V_{H}.

4. The protein according to any one of the preceding claims, wherein the linker comprises the sequence set forth in sequence ID No. 1.

5. The protein according to any one of the preceding claims, wherein the linker is free of charged amino acid residues.

6. The protein according to any one of the preceding claims, wherein the linker consists of serine and glycine amino acid residues.

7. The protein according to any one of the preceding claims, wherein the linker comprises at least approximately 75% serine residues.

8. The protein according to claim 2 or any claim dependent thereon, wherein y is any integer selected to optimise the biological function and three dimensional conformation of the biosynthetic protein.

9. A method for producing a fusion protein, comprising:
transforming a cell with a DNA construct encoding the protein according to any one of the preceding claims;
inducing the transformed cell to express said fusion protein; and
collecting said expressed fusion protein.

10. A DNA encoding the protein according to any one of the preceding claims.

11. A cell which expresses the DNA of claim 10.

12. The protein of claim 9 wherein the linker region comprises threonine.

13. The protein of claim 9, wherein at least 75% of the residues are serine.

## Patentansprüche

1. Ein biosynthetisches Protein aufweisend erste und zweite Proteindomänen, die enweder individuell oder zusammen biologisch aktiv sind, wobei die Domänen durch einen Peptidlinker verbunden sind, der aus zwischen 8 und 40 Aminosäureresten besteht, wovon mindestens 50% der Reste Serin sind, wobei Ser Ile (Ser Ser Ser Ser Gly)₃ Ser Asp Ile ausgeschlossen ist.

2. Das biosynthetische Protein gemäß Anspruch 1, wobei der Peptidlinker die Sequenz (Ser Ser Ser Ser Gly)_{y} aufweist, wobei y größer als 1 ist.

3. Das Protein gemäß Anspruch 1 oder 2, wobei:
(i) eine der Proteindomänen einen variablen Bereich der schweren Kette eines Antikörpers (VH) aufweist, und die andere der Proteindomänen einen variablen Bereich der leichten Kette eines Antikörpers (VL) aufweist, wobei das Protein wahlweise mit einem radioaktiven Isotop markiert ist, oder
(ii) die erste Proteindomäne einen Polypeptidligand aufweist, und die zweite Domäne einen Polypeptideffektor aufweist, wobei der Ligand imstande ist, einen Rezeptor oder ein auf einer Zelle sich befindendes Adhäsionsmolekül zu binden, und der Effektor imstande ist, den Stoffwechsel der Zelle zu beeinflussen, und zum Beispiel der Ligand ein sFv Fusionsprotein oder ein Antikörperfragment ist und zum Beispiel der Effektor ein Toxin ist; oder
(iii) eine der Proteindomänen die Struktur eines V_{L} hat und die andere der Proteindomänen die Struktur eines V_{H} hat.

4. Das Protein gemäß einem der vorangehenden Ansprüche, wobei der Linker die in Sequenz ID NO. 1 angegebene Sequenz aufweist.

5. Das Protein gemäß einem der vorangehenden Ansprüche, wobei der Linker frei von geladenen Aminosäureresten ist.

6. Das Protein gemäß einem der vorangehenden Ansprüche, wobei der Linker aus Serin und Glycin Aminosäureresten besteht.

7. Das Protein gemäß einem der vorangehenden Ansprüche, wobei der Linker mindestens etwa 75% Serinreste aufweist.

8. Das Protein gemäß Anspruch 2 oder irgendeinem davon abhängigen Anspruch, wobei y irgendeine Integralzahl ist, die so ausgewählt ist, um die biologische Funktion und die dreidimensionale Konformation des biosynthetischen Proteins zu optimieren.

9. Ein Verfahren zur Herstellung eines Fusionsproteins, aufweisend:
Transformieren einer Zelle mit einem DNA-Konstrukt, der das Protein gemäß einem der vorangehenden Ansprüche encodiert;
Induzieren der transformierten Zelle, um das Fusionsprotein zu exprimieren; und
Gewinnen des exprimierten Fusionsproteins.

10. Eine DNA, die das Protein gemäß einem der vorangehenden Ansprüche encodiert.

11. Eine Zelle, die die DNA gemäß Anspruch 10 exprimiert.

12. Das Protein gemäß Anspruch 9, wobei der Linkerbereich Threonin aufweist.

13. Das Protein gemäß Anspruch 9, wobei mindestens 75% der Reste Serin sind.

## Revendications

1. Protéine biosynthétique comprenant un premier et second domaines protéiques biologiquement actifs individuellement ou ensemble, lesdits domaines étant reliés par un segment de liaison peptidique constitué de entre 8 et 40 résidus d'acides aminés, dont au moins 50% des résidus sont une sérine, à l'exclusion de Ser Ile( Ser Ser Ser Ser Gly)₃ Ser Asp Ile.

2. Protéine biosynthétique selon la revendication 1, dans laquelle le segment de liaison peptidique comprend la séquence (Ser Ser Ser Ser Gly)_{y} où y est supérieur à 1.

3. Protéine selon la revendication 1 ou 2 dans laquelle :
(i) un desdits domaines protéiques comprend une région variable de chaîne lourde d'anticorps (VH) et l'autre desdits domaines protéique comprend une région variable de chaîne légère d'anticorps (VL), ladite protéine étant éventuellement marquée avec un isotope radioactif ; ou
(ii) le premier domaine protéique comprend un ligand polypeptidique et le second domaine comprend un effecteur polypeptidique, ledit ligand étant capable de se lier à un récepteur ou une molécule d'adhésion sur une cellule et ledit effecteur étant capable d'affecter le métabolisme de la cellule, et par exemple le ligand est une protéine de fusion sFv, ou un fragment d'anticorps, et par exemple l'effecteur est une toxine ; ou
(iii) l'un desdits domaines protéiques a la structure d'un V_{L} et l'autre desdits domaines protéiques a la structure d'un V_{H}.

4. Protéine selon l'une quelconque des revendications précédentes, dans laquelle ledit segment de liaison comprend la séquence représentée dans SEQ ID NO: 1.

5. Protéine selon l'une quelconque des revendications précédentes, dans laquelle ledit segment de liaison est dépourvu de résidus d'acides aminés chargés.

6. Protéine selon l'une quelconque des revendications précédentes, dans laquelle ledit segment de liaison est constitué de résidus d'acides aminés sérine et glycine.

7. Protéine selon l'une quelconque des revendications précédentes, dans laquelle ledit segment de liaison comprend au moins environ 75 % de résidus sérine.

8. Protéine selon la revendication 2 ou toute revendication dépendante de celle-ci, dans laquelle y est un nombre entier choisi pour optimiser la fonction biologique et la conformation tridimensionnelle de la protéine biosynthétique.

9. Méthode de production d'une protéine de fusion, comprenant :
la transformation d'une cellule avec un construit d'ADN codant pour la protéine selon l'une quelconque des revendications précédentes ;
l'induction de la cellule transformée pour exprimer ladite protéine de fusion ; et
la récolte de ladite protéine de fusion exprimée.

10. ADN codant pour la protéine selon l'une quelconque des revendications précédentes.

11. Cellule exprimant l'ADN de la revendication 10.

12. Protéine selon la revendication 9, dans laquelle ledit segment de liaison comprend la thréonine.

13. Protéine selon la revendication 9, dans laquelle au moins 75 % des résidus sont la sérine.
